# NEUE EUROPÄISCHE PATENTSCHRIFT

(11) **EP 0 925 272 B2**
(45) Veröffentlichungstag und Bekanntmachung des Hinweises auf die Entscheidung über den Einspruch: **28.12.2011**
(45) Hinweis auf die Patenterteilung: 12.09.2001
(21) Anmeldenummer: 97928252.2
(22) Anmeldetag: 19.06.1997
(51) Int. Cl.: C07C 57/04, F23G 5/00

(54) **VERFAHREN ZUR ENTSORGUNG VON BEI DER ACRYLSÄURE- ODER METHACRYLSÄURE-HERSTELLUNG ANFALLENDEN NEBENKOMPONENTEN**
METHOD FOR REMOVING BY-PRODUCTS OBTAINED WHEN PRODUCING ACRYLIC ACID OR METHACRYLIC ACIDS
PROCEDE POUR L'ELIMINATION DE SOUS-PRODUITS SE PRESENTANT LORS DE LA FABRICATION D'ACIDE ACRYLIQUE OU D'ACIDE METHACRYLIQUE

(30) Priorität: 20.06.1996 DE 19624674
(43) Veröffentlichungstag der Anmeldung: 30.06.1999
(73) Patentinhaber: BASF SE, 67056 Ludwigshafen (DE)
(72) Erfinder: SCHLIEPHAKE, Volker, D-67105 Schifferstadt (DE); HAMMON, Ulrich, D-68165 Mannheim (DE); PIES, Wolfgang, D-67227 Frankenthal (DE); RAUH, Ulrich, D-67117 Limburgerhof (DE)
(74) Vertreter: Isenbruck, Günter
(86) Internationale Anmeldenummer: PCT/EP1997/003216
(87) Internationale Veröffentlichungsnummer: WO 1997/048669

(56) Entgegenhaltungen:
- FR-A- 2 722 868
- US-A- 4 255 590
- US-A- 5 005 493
- KIRK-OTHMER: "ENCYCLOPEDIA OF CHEMICAL TECHNOLOGY, VOL.13" 1989 , JOHN WILEY & SONS , NEW YORK-CHICHESTER-BRISBANE-TORONTO XP002042165 349 siehe Seite 182 - Seite 206 siehe Seite 191, Zeile 12 - Zeile 23 siehe Seite 193; Tabelle 5 siehe Seite 196, Zeile 1 - Zeile 33 siehe Seite 198, Zeile 16 - Zeile 29 siehe Seite 199; Abbildung 4 siehe Seite 200, Zeile 27 - Zeile 40

## Beschreibung

Die vorliegende Erfindung betrifft ein Verfahren zur Entsorgung von bei der Acrylsäure- oder Methacrylsäure-Herstellung anfallenden leicht-, mittel-, und schwersiedenden Nebenkomponenten.

Acrylsäure ist eine bedeutende Grundchemikalie. Aufgrund ihrer sehr reaktionsfähigen Doppelbindung sowie der Säurefunktion eignet sie sich insbesondere als Monomeres zur Herstellung von Polymerisaten. Von der hergestellten Menge an Acrylsäuremonomeren wird der größere Teil vor der Polymerisation - zu z.B. Klebstoffen, Dispersionen oder Lacken -verestert. Nur der kleinere Teil der hergestellten Acrylsäuremonomeren wird direktzu z.B. "Superabsorbern" - polymerisiert. Während im allgemeinen bei der direkten Polymerisation der Acrylsäure Monomere hoher Reinheit benötigt werden, sind die Anforderungen an die Reinheit der Acrylsäure nicht so hoch, wenn diese vor der Polymerisation verestert wird.

Es ist allgemein bekannt, daß Acrylsäure durch heterogen katalysierte Gasphasenoxidation von Propen mit molekularem Sauerstoff an im festen Aggregatzustand befindlichen Katalysatoren bei Temperaturen zwischen 200 und 400°C zweistufig über Acrolein hergestellt werden kann (vgl. z.B. DE-A-1 962 431, DE-A-2 943 707, DE-C-1 205 502, EP-A-0 257 565, EP-A-0 253 409, DE-A 2 251 364, EP-A-0 117 146, GB-B-1 450 986 und EP-A-0 293 224). Hierbei werden oxidische Mehrkomponenten-Katalysatoren z.B. auf der Basis von Oxiden der Elemente Molybdän, Chrom, Vanadium oder Tellur eingesetzt.

Aus DE-C-2 136 396 ist bekannt, die Acrylsäure aus den bei der katalytischen Oxidation von Propen bzw. Acrolein erhaltenen Reaktionsgasen durch Gegenstromabsorption mit einem Gemisch aus 75 Gew.-% Diphenylether und 25 Gew.-% Diphenyl abzutrennen. Weiterhin ist aus DE-A-2 449 780 das Abkühlen des heißen Reaktionsgases durch Teilverdampfen des Lösungsmittels in einem Direktkondensator (Quenchapparat) vor der Gegenstromabsorption bekannt. Problematisch ist hierbei sowie bei weiteren Verfahrensschritten der Anfall von Feststoffen in den Apparaten, der die Anlagenverfügbarkeit reduziert. Gemäß DE-A-4 308 087 kann dieser Feststoffanfall dadurch reduziert werden, indem man dem relativ unpolaren Lösungsmittelgemisch aus Diphenylether und Diphenyl (Diphyl) ein polares Lösungsmittel, wie Dimethylphthalat in einer Menge von 0,1 bis 25 Gew.-% zufügt.

Neben der oben beschriebenen Absorption des die Acrylsäure enthaltenden Reaktionsprodukts in ein hochsiedendes Lösungsmittel(gemisch) existieren noch andere Verfahrensprinzipien. Das oben beschriebene Verfahren unterscheidet sich von den anderen Verfahren dadurch, daß hier die Acrylsäure möglichst wasserfrei in ein hochsiedendes Lösungsmittel(gemisch) absorbiert wird. Das Wasser wird in einem separaten Verfahrensabschnitt aus dem Verfahren abgezogen. Die anderen Verfahren sehen eine Totalkondensation von Acrylsäure und des weiterhin bei der katalytischen Oxidation entstehenden Reaktionswassers vor. Dabei entsteht eine wäßrige Acrylsäurelösung, die über Destillation mit einem Mittel, das mit Acrylsäure ein Azeotrop bildet (vgl. DE-C-3 429 391, JP-A-1 124 766, JP-A-7 118 766, JP-A-7 118 966, JP-A-7 118 968 und JP-A-7 241 885), oder über ein Extraktionsverfahren (vgl. DE-A-2 164 767, JP-A-5 8140 039 und JP-4 8091 013) weiter aufgearbeitet werden kann. In EP-A-0 551 111 wird das mittels katalytischer Gasphasenoxidation hergestellte Gemisch von Acrylsäure und Nebenprodukten mit Wasser in einem Absorptionsturm in Berührung gebracht und die erhaltene wä rige Lösung in Anwesenheit eines Lösungsmittels, das mit polaren Leichtsiedern wie Wasser oder Essigsäure ein Azeotrop bildet, destilliert. DE-C-2 323 328 beschreibt die Abtrennung von Acrylsäure aus einer wä rigen Acrylsäure-Veresterungsablauge oder einer wä rigen Acrlysäure-Lösung, wie sie bei der Acrylsäure-Herstellung durch Oxidation von Propen oder Acrolein entsteht, durch Extraktion mit einem speziellen Gemisch organischer Lösungsmittel.

Allen industriell genutzten Verfahren zur Herstellung von Acrylsäure oder Methacrylsäure ist gemeinsam, daß Tief-, Mittel- und Hochsieder als unerwünschte Nebenkomponenten und damit als zu entsorgende Stoffströme anfallen. Diese mindestens drei Stoffströme stellen eine erhebliche Kostenbelastung für das Verfahren dar.

Somit bestand die Aufgabe der vorliegenden Erfindung darin, ein Entsorgungsverfahren zu schaffen, das das Nebenproduktproblem der Acrylsäure- und Methacrylsäureherstellung ökonomisch und ökologisch auf möglichst optimale Weise löst.

Überraschenderweise wurde gefunden, daß sich dieses Problem lösen läßt durch gemeinsame Verbrennung der gasförmigen Leichtsieder mit dem wäßrig-flüssigen Stoffstrom von wasserlöslichen Leicht- und/oder Mittelsiedern, wobei die Hochsieder ggf. mitverbrannt werden.

In Kirk-Othmer, "Encyclopedia of Chemical Technology", Vol. 13, 1989, S. 182-206, wird zwar allgemein die Verbrennung von festen, flüssigen und fest/flüssigen Industrieabfällen beschrieben, und es werden die hierfür verwendeten Öfen vorgestellt. Es findet sich jedoch kein Hinweis darauf, das sich bei der (Meth)acrylsäureherstellung ergebende Nebenproduktproblem durch die gemeinsame Verbrennung der gasförmigen Leichtsieder mit dem genannten wäßrig-flüssigen Stoffstrom zu lösen.

Somit betrifft die Erfindung ein Verfahren zur Entsorgung von bei der Acrylsäure- oder Methacrylsäure-Herstellung anfallenden Nebenkomponenten, bei dem gasförmige leichtsiedende Nebenkomponenten verbrannt werden, wobei in Wasser gelöste leicht- oder mittelsiedende Nebenkomponenten in die Verbrennungsstufe der Nebenkomponenten zugeführt werden, wobei die leichtsiedenden Nebenkomponenten einen niedrigeren Siedepunkt als die Acrylsäure oder Methacrylsäure besitzen und die mittelsiedenden Nebenkomponenten in etwa den gleichen Siedepunkt wie die Acrylsäure oder Methacrylsäure besitzen, wobei die Acrylsäure oder Methacrylsäure hergestellt wird durch
(a) katalytische Gasphasenoxidation von Propen oder Isobuten und/oder Acrolein oder Methacrolein,
(b) Absorption des in Stufe (a) entstehenden Reaktionsprodukts in einem Lösungsmittel und
(c) Abtrennung der Acrylsäure oder Methacrylsäure aus dem beladenen Lösungsmittel der Stufe (b) durch Extraktion und/oder Destillation,
wobei vor der Absorption in Stufe (b) ein Teil des Lösungsmittels verdampft wird und von dem verbleibenden flüssigen Lösungsmittel ein Teil, ggf. nach Lösungsmittelverdampfung, als schwersiedende Nebenkomponente entsorgt wird, und
nach der Absorption des Reaktionsprodukts in Stufe (b) verbleibendes, nicht absorbiertes Reaktionsprodukt abgekühlt wird, wobei das erhaltene wäßrige Kondensat, ggf. nach anschließender Extraktion, als Nebenkomponente und wenigstens ein Teil des erhaltenen Gasstroms als Nebenkomponente entsorgt werden. Zweckmäßigerweise erfolgt die Verbrennung in einer Brennkammer mit einem oder mehreren Stützgas-, Düsen- oder Mehrstoffbrennern, z. B., einem Flammenverdampfungsbrenner.

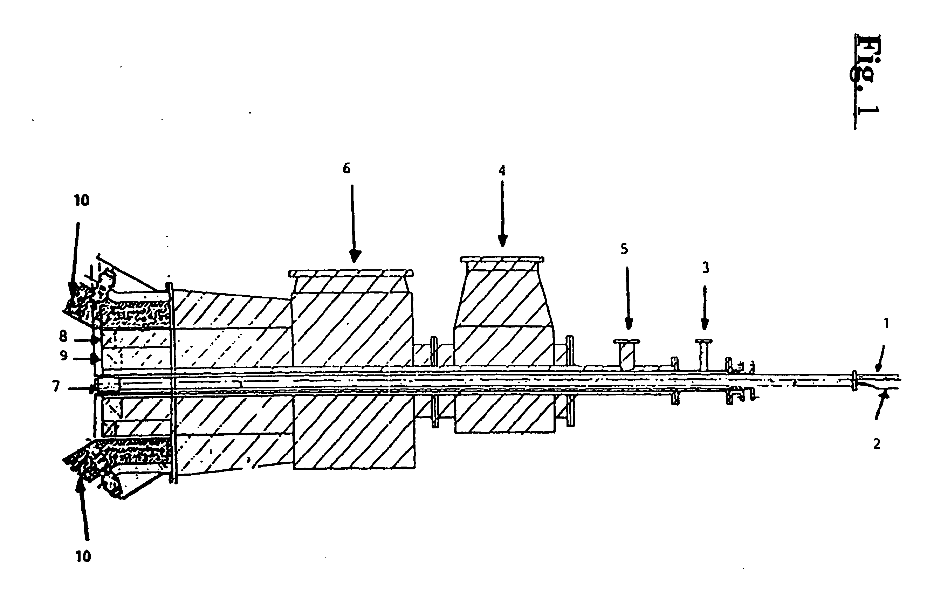

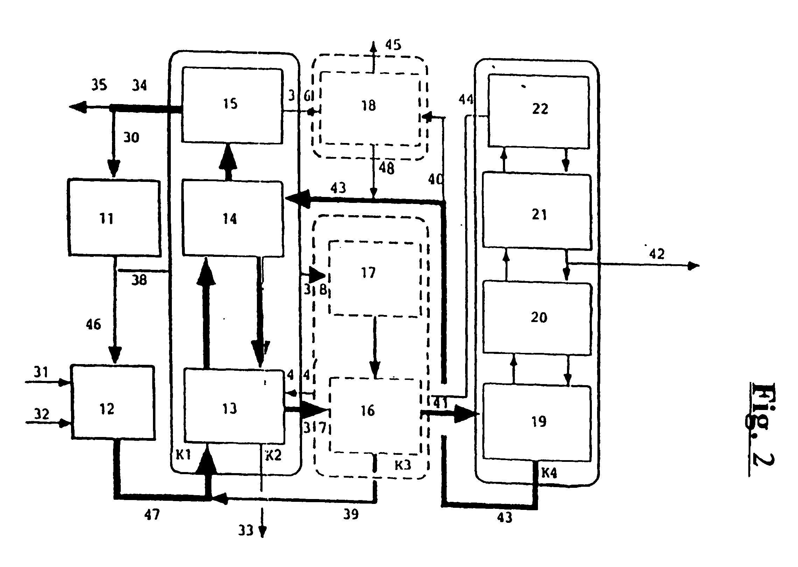

Bevorzugte Ausführungsformen der Erfindung sind in den Unteransprüchen definiert. Weitere und bevorzugte Merkmale sind in den Figuren 1 und 2 sowie der Beschreibung gezeigt.

### In den Zeichnungen zeigen:

- Fig. 1: schematische Darstellung eines bei Durchführung des erfindungsgemäßen Verfahrens bevorzugt eingesetzten Flammenverdampfungsbrenners;
- Fig. 2: ein Verfahrensschema zur Herstellung von Acrylsäure.

Vorliegend bezeichnen die Begriffe Hoch- oder Schwersieder, Mittelsieder und Tief- oder Leichtsieder sowie entsprechend adjektivisch gebrauchte Begriffe Verbindungen, die einen höheren Siedepunkt als die Acrylsäure bzw. Methacrylsäure besitzen (Hochsieder) bzw. solche, die in etwa den gleichen Siedepunkt wie Acrylsäure bzw. Methacrylsäure besitzen (Mittelsieder) bzw. solche, die einen niedrigeren Siedepunkt besitzen (Leichtsieder).

Die gasförmigen leichtsiedenden Nebenkomponenten (1), die neben bei der Säureherstellung anfallenden gasförmigen Nebenkomponenten auch tiefsiedende (bei Raumtemperatur) flüssige Komponenten umfassen, enthalten im wesentlichen neben inerten Gasen, wie Stickstoff, Wasser Kohlenmonoxid, Kohlendioxid, die Leichtsiederfraktion der zu entsorgenden Stoffe, vorzugsweise Acrolein, Acetaldehyd, Propan, Propen, Formaldehyd, Ameisensäure und/oder bei der Acrylsäure- oder Methacrylsäure-Herstellung nicht umgesetzte Edukte. Dieser Strom (1) wird vorteilhafterweise in einer Brennkammer verbrannt. Eine solche Brennkammer kann entweder der Acrylsäure- oder Methacrylsäureproduktionsanlage allein zugeordnet sein oder im Verbund mit anderen Anlagen betrieben werden. Ein Beispiel für einen bevorzugt eingesetzten Flammenverdampfungsbrenner ist in der unten näher beschriebenen Figur 1 gezeigt.

Die zu entsorgenden, in Wasser gelösten leicht- oder mittelsiedenden Nebenkomponenten (2) enthalten vorzugsweise 0,1 bis 10 Gew.-% Essigsäure, 0,01 bis 5 Gew.-% Maleinsäure, 0,01 bis 8 Gew.-% Fumarsäure, 0,2 bis 4 Gew.-% Formaldehyd und/oder 0,1 bis 10, vorzugsweise 5 Gew.-%, und sonstiges organisches Material wie Ameisensäure, Propionsäure, Benzaldehyd, Diacrylsäure, Hydroxypropionsäure, Benzoesäure und/oder Diphenylether (Reste davon, wenn als Lösungsmittel eingesetzt), jeweils bezogen auf 100 Gew.-% der Nebenkomponenten (2). Überwiegend enthalten diese Nebenkomponenten Essigsäure, Maleinsäure und Formaldehyd. Dieser Nebenkomponentenstrom (2) wird bei der Verbrennung der leichtsiedenden Nebenkomponenten (1) als wäßriger Stoffstrom zugeführt und vorzugsweise eingedüst. In einer vorteilhaften Ausführungsform der Erfindung erfolgt dieses Eindüsen über konzentrische Mehrstoffdüsen, wie sie Stand der Technik sind. Vorteilhafterweise wird dieser wäßrige, die Nebenkomponenten (2) enthaltende Strom so weit wie möglich vorkonzentriert, z.B. durch Eindampfen. Hierzu kann zweckmäßigerweise im Sinne eines Energieverbundes die im Produktionsprozeß anfallende Wärme genutzt werden. Als Apparate eignen sich handelsübliche Verdampfer.

Die schwersiedenden Nebenkomponenten (3) enthalten überwiegend Acrylsäure- bzw. Methacrylsäure-Polymerisate, nicht verbrauchte Produktionsstabilisatoren, z.B. Phenothiazin oder p-Nitrosophenol, Inhibitoren, z.B. Methylenblau, und/oder deren thermische Abbauprodukte.

Dieser Schwersieder-Feststoffstrom (3) wird vorteilhafterweise zunächst vorbehandelt, da ihn seine physikalisch-chemischen Eigenschaften schwer handhabbar machen. So liegt der Stockpunkt im allgemeinen so hoch, daß dieser Stoffstrom bei üblichen Umgebungstemperaturen nicht pumpfähig ist. Eine Abmischung mit einem bei Umgebungstemperatur niedrigviskosen Lösungsmittel (Viskosität üblicherweise von 0,5 • 10⁻³ bis 2 • 10⁻³ Pa • s) von vorzugsweise hohem Siedepunkt, d.h. von 100 bis 250 °C, kann den Stockpunkt so weit senken, daß die Mischung pumpfähig wird. Besonders bewährt haben sich in diesem Zusammenhang Aceton, Dimethylformamid (DMF) oder Alkohole, insbesondere C₃-C₁₀-Diole und/oder -Triole, wie z.B. Pentandiol, Hexandiol, Glycerin, Glykol, Dimethylglykol, Monomethylglykol, Monoethylglykol, Diethylglykol oder Mischungen davon. Der mit solchen niedrigviskosen Verdünnungsmitteln behandelte Nebenkomponenten-Stoffstrom (3) kann der Verbrennung gemäß dem erfindungsgemäßen Verfahren zugeführt werden, wobei die Zuführung getrennt von den anderen Nebenkomponentenströmen (1) und (2) erfolgt. Vorzugsweise werden die Nebenkomponenten (2) und (3) getrennt eingedüst. Daneben besteht auch die Möglichkeit, die schwersiedenden Nebenkomponenten (3) nach entsprechender Aufarbeitung einer industriellen Reststoffverbrennungsanlage, wie einer dezentralen Vorort-Verbrennung, zu übergeben, wo sie von ihren Brenneigenschaften schwerem Heizöl vergleichbar sind, oder anderweitig einer Weiterverarbeitung zuzuführen.

Figur 1 zeigt einen Querschnitt durch einen bei Durchführung des erfindungsgemäßen Verfahrens bevorzugt eingesetzten Brenner vom Typ Flammenverdampfungsbrenner. Die wesentlichen Teile dieses Brenners sind mit Bezugszeichen versehen. Hierbei bezeichnen die Bezugsziffern 1 bis 5 Zufuhrleitungen wie folgt: Zufuhrleitung 1 für Abwasser, Zufuhrleitung 2 für Zerstäubungsdampf für das Abwasser, Zufuhrleitung 3 für flüssigen oder gasförmigen (z.B. Erdgas und/oder CO) Zusatzbrennstoff, Zufuhrleitung 4 für Nebenkomponentenstrom (2) und Zufuhrleitung 5 für Stützgas, wie Erdgas. Über die Zufuhrleitung 6 wird Verbrennungsluft eingeführt. Bei Zufuhrleitung 6 wird getrennt auch der Nebenkomponentenstrom (1) zu-geführt. Dieser Nebenkomponentenstrom wird separat zur Verbrennungsluft zugeführt, um eine Explosion zu vermeiden. Über die Düse 7 werden die flüssigen Komponenten in die Brennkammer eingesprüht. Bezugsziffer 8 und 9 bezeichnen Drallringe, die zur Luftführung bzw. Abgasführung dienen. Bezugsziffer 10 bezeichnet feuerfestes Isoliermaterial. Bei der Düse 7 kann es sich um eine bekannte konzentrische Mehrstoffdüse handeln, die mehrere zentrale Zuführungsringspalte aufweist, die zur Zuführung von Nebenkomponentenstrom (2), Zerstäuberdampf für diesen Nebenkomponentenstrom, sowie die weiteren oben angegebenen Komponenten geeignet sind. Somit werden mit Ausnahme der Verbrennungsluft und des Nebenkomponentenstroms (1) alle Komponenten über die Düse zugeführt. Wird der Nebenkomponentenstrom (3) (in handhabbarer Form) ebenfalls eingedüst, so muß die Düse 7 einen weiteren zentralen Zuführungsringspalt aufweisen.

Die Verbrennung selbst findet in einer Brennkammer (nicht gezeigt) bei adiabaten Verbrennungstemperaturen von 800 bis 1200 °C, vorzugsweise von 800 bis 1000 °C, statt. In Fig. 1 wäre die Brennkammer, deren Beginn das feuerfeste Isoliermaterial 10 markiert, links von Düse 7. Vorteilhafterweise ist die Brennkammer entweder der Acrylsäure- oder Methacrylsäure-Produktionsanlage allein zugeordnet, oder sie wird im Verbund mit anderen Anlagen betrieben. Die hierbei freiwerdende Energie kann in einem von der Brennkammer getrennt angeordneten Abhitzeteil günstig, z.B. in Form von hochgespanntem Dampf, wiedergewonnen werden. Die Verbrennungsgase werden im Abhitzeteil zweckmäßigerweise so geführt, daß die Temperaturen von unten nach oben sukzessive abnehmen. So kommen die Gase üblicherweise mit einer Verbrennungstemperatur von 850 bis 1000 °C, vorzugsweise etwa 860 °C, im Abhitzeteil an und geben dann an Wärmeaustauschern, die der Rückgewinnung der Energie dienen, einen Teil ihrer Energie durch Abkühlung ab. Die Temperatur am Auslaß des Abhitzeteils beträgt dann etwa 200 °C. Der die Nebenkomponenten (2) enthaltende eingedüste wäßrige Stoffstrom trägt je nach Gehalt an brennbaren Stoffen einen positiven oder negativen Beitrag zur Energieausbeute in der Brennkammer bei, da die Verdampfungsenthalpie des Wassers zunächst als Energie in das System hineingesteckt werden muß. Deshalb ist es auch vorteilhaft, diesen Strom so weit wie möglich vorzukonzentrieren. Die bei der Verbrennung entstehende Abwärme wird vorteilhafterweise zur Dampferzeugung, z.B. von Wasserdampf, genutzt.

Die Erfindung betrifft ein Verfahren, in dem die Stufen (a) bis (c) wie oben definiert sind. Diese einzelnen Stufen werden im folgenden für Acrylsäure beschrieben. Sie gelten analog für Methacrylsäure, soweit nichts anderes angegeben ist. Ein geeignetes Verfahrensschema, in dem die Stufen (a) bis (c) sowie die zu entsorgenden Nebenkomponentenströme (1) bis (3) gezeigt sind, stellt Fig. 2 dar. In Fig. 2 bezeichnen die Bezugsziffern 11 bis 22 Verfahrensstufen, die Bezugsziffern 30 bis 48 bezeichnen Zu- oder Abfuhrleitungen und K1 bis K4 bezeichnen verschiedene Kolonnen/Apparate.

### Stufe (a):

Stufe (a) umfaßt die ein- bzw. zweistufige katalytische Gasphasenreaktion von Propen und/oder Acrolein mit molekularem Sauerstoff zu Acrylsäure. Bei Methacrylsäure erfolgt analog eine Gasphasenreaktion von Isobuten und/oder Methacrolein mit molekularem Sauerstoff. Die Gasphasenreaktion kann nach bekannten Verfahren, insbesondere wie sie in den oben genannten Druckschriften beschrieben sind, erfolgen. Vorteilhafterweise wird hierbei bei Temperaturen zwischen 200 und 500°C gearbeitet. Vorzugsweise werden als heterogene Katalysatoren oxidische Mehrkomponenten-Katalysatoren auf der Basis der Oxide von Molybdän, Chrom, Vanadium und/oder Tellur eingesetzt. Außerdem besteht auch die Möglichkeit, Methacrylsäure zweistufig herzustellen durch (1) Kondensation von Propionaldehyd mit Formaldehyd (in Gegenwart eines sekundären Amins als Katalysator) zu Methacrolein und (2) anschließende Oxidation des Methacroleins zu Methacrylsäure.

Gemäß der in Fig. 2 gezeigten bevorzugten Ausführungsform wird nach der Verdichtung 11 des Kreisgases aus Leitung 30, das im wesentlichen aus Stickstoff, Kohlenoxiden und nicht umgesetzten Edukten besteht, dieses über Leitung 46 zusammen mit Propen aus Leitung 31 und Luft aus Leitung 32 einem Reaktor zugeführt, in dem die heterogen katalysierte Oxidation (Synthese) 12 zu Acrylsäure stattfindet.

In Stufe (a) wird jedoch nicht reine Acrylsäure, sondern ein gasförmiges Gemisch erhalten, das Acrylsäure und als Nebenkomponenten im wesentlichen nicht umgesetztes Acrolein und/oder Propen, Wasserdampf, Kohlenmonoxid, Kohlendioxid, Stickstoff, Sauerstoff, Essigsäure, Propionsäure Formaldehyd, weitere Aldehyde und Maleinsäureanhydrid enthalten kann. Insbesondere enthält das Reaktionsproduktgemisch typischerweise, jeweils angegeben in Gew.-% bezogen auf das gesamte Reaktionsgemisch, bis 1 % Propen und bis 1 % Acrolein, bis 2 % Propan, bis 20 % Wasserdampf, bis 15 % Kohlenoxide, bis 90 % Stickstoff, bis 5 % Sauerstoff, bis 2 % Essigsäure, bis 2 % Propionsäure, bis 1 % Formaldehyd, bis 2 % Aldehyde sowie bis 0,5 % Maleinsäureanhydrid.

### Stufe (b)

In Stufe (b) werden die Acrylsäure und ein Teil der Nebenkomponenten aus dem Reaktionsgas durch Absorption mit einem hochsiedenden Lösungsmittel abgetrennt. Hierfür geeignet sind alle hochsiedenden Lösungsmittel, die Acrylsäure absorbieren, insbesondere Lösungsmittel mit einem Siedepunkt über 160 °C. Besonders geeignet ist ein Gemisch aus Diphenylether und Biphenyl, wie z. B. das im Handel erhältliche Gemisch aus 75 Gew.-% Diphenylether und 25 Gew.-% Biphenyl, das auch Diphyl genannt wird.

Vorteilhafterweise wird gemäß Fig. 2 das aus Stufe (a) nach Synthese 12 erhaltene heiße Reaktionsgas über Leitung 47 einem Quenchapparat oder Direktkondensator K1 zugeführt, in dem es durch Teilverdampfen 13 des Lösungsmittels vor der Absorption abgekühlt wird. Für K1 eignen sich insbesondere Venturiwäscher, Blasensäulen oder Sprühkondensatoren. Dabei kondensieren die schwersiedenden Nebenkomponenten des Reaktionsgases aus Stufe (a) in das nicht verdampfte Lösungsmittel (in Fig. 2 ist die Kondensation und Teilverdampfung mit Bezugsziffer 13 bezeichnet). Außerdem ist die Teilverdampfung des Lösungsmittels ein Reinigungsschritt für das Lösungsmittel. In einer bevorzugten Ausführungsform der Erfindung wird ein Teilstrom des nicht verdampften Lösungsmittels, vorzugsweise 1 bis 10 % des der Absorptionskolonne zugeführten Massenstroms, aus dem Kondensator K1 abgezogen und einer Lösungsmittelreinigung unterworfen (nicht gezeigt). Hierbei wird das Lösungsmittel überdestilliert. Die Lösungsmitteldestillation dient der Vermeidung einer Aufpegelung an ungewollten Lösungsmittelinhaltsstoffen. Die schwersiedenden Nebenkomponenten, die vornehmlich undefinierte Acrylsäurepolymerisate und Abbauprodukte der eingesetzten Stabilisatoren enthalten, bleiben zurück und werden als Nebenkomponenten (3) über Leitung 33 entsorgt.

Die Absorption 14 erfolgt in einer Gegenstromabsorptionskolonne K2, die vorzugsweise mit Sieb-, Ventil- oder Dualflowböden bestückt ist, die von oben mit (nicht verdampftem) Lösungsmittel beaufschlagt werden. Das gasförmige Reaktionsprodukt und gegebenenfalls verdampftes Lösungsmittel werden von unten in die Kolonne K2 eingeleitet und anschließend auf Absorptionstemperatur abgekühlt. Im Absorptionsteil der Kolonne K1 werden aus der Acrylsäure sowohl mittel- wie auch schwersiedende Komponenten nahezu vollständig aus dem Gasreststrom entfernt.

Das verbleibende, nicht absorbierte Reaktionsgas von Stufe (a) wird weiter abgekühlt, um den kondensierbaren Teil der leichtsiedenden Nebenkomponenten davon, insbesondere Wasser, Formaldehyd und Essigsäure, durch Kondensation, in Fig. 2 als Sauerwasser-Quench 15 gezeigt, abzutrennen. Dieses Kondensat, das über Leitung 36 abgezogen wird, wird deshalb im folgenden Sauerwasser genannt. Der verbleibende Gasstrom, im folgenden Kreisgas genannt, besteht überwiegend aus Stickstoff, Kohlenoxiden und nicht umgesetzten Edukten. Vorzugsweise wird dieser teilweise wieder als Verdünnungsgas den Reaktionsstufen zugeführt. Das Kreisgas verläßt über Leitung 34 die Absorptionskolonne K2 über Kopf. Dort wird der Strom geteilt, vorzugsweise im Verhältnis 3:1 bis 1:3 je nach Bauart, insbesondere 1:1, wobei der eine Teil über Leitung 30 über einen Kreisgasverdichter (in Fig. 2 als Kreisgasverdichtung 11 gezeigt) in die Reaktoren zur Synthese 12 zurückgeführt wird und der zweite Teilstrom als zu entsorgender gasförmiger Nebenkomponentenstrom (1) über Leitung 35 ausgeschleust wird. Das o.g. Sauerwasser stellt die zu entsorgenden in Wasser gelösten Nebenkomponenten (2) dar. Dieses besteht aus dem in Stufe (a) gebildeten Wasser sowie wasserlöslichen Leicht- und Mittelsiederkomponenten, wie z.B. Essigsäure, Formaldehyd, Maleinsäure etc.

Aus dem Absorptionsteil der in Stufe (b) eingesetzten Kolonne K2 wird ein mit Acrylsäure, schwer- und mittelsiedenden Nebenkomponenten sowie einem geringen Anteil an leichtsiedenden Nebenkomponenten beladener Lösungsmittelstrom über Leitung 37 abgezogen und in einer bevorzugten Ausgestaltung der Erfindung einer Desorption mit Leichtsiederstrippung 16 und Leichtsiederwäsche 17 unterzogen. Vorteilhafterweise wird diese in einer Desorptionskolonne K3 gemäß Fig. 2, die vorzugsweise mit Sieb-, Ventiloder Dualflowböden aber auch mit Füllkörpern oder geordneten Packungen bestückt sein kann, in Anwesenheit eines sog. Stripp- oder Abstreifgases durchgeführt. Bei dem Strippgas kann jedes inerte Gas oder Gasgemisch verwendet werden, vorzugsweise wird ein Gasgemisch von Luft und Stickstoff verwendet oder ein Teilstrom des in (a) beschriebenen Kreisgases. Bei der Desorption 16 und 17 wird der größte Teil der Leichtsieder mit einem Teil des Kreisgases, das vor Stufe (a) über Leitung 38 entnommen wird, aus dem beladenen Lösungsmittel gestrippt und in Kolonne K2 über Leitung 39 zurückgeführt.

Aus dem Sumpf der Desorptionskolonne K3 wird ein nahezu leichtsiederfreier, mit Acrylsäure beladener Lösungsmittelstrom über Leitung 41 abgezogen und der Kolonne K4 zur Destillation zugeführt.

### Stufe (c):

In Verfahrensstufe (c) wird die Acrylsäure zusammen mit den mittelsiedenden Komponenten sowie dem letzten Rest an leichtsiedenden Nebenkomponenten vom Lösungsmittel abgetrennt. Diese Trennung erfolgt mittels Destillation, wobei grundsätzlich jede Destillationskolonne verwendet werden kann. Vorteilhafterweise wird hierzu eine Kolonne mit Siebböden, Dual-Flow-Böden oder Querstromsiebböden aus Metall oder mit Ventilböden verwendet. Die Destillation ist in Fig. 2 gezeigt mit den einzelnen Verfahrensstufen Lösungsmittelgemischdestillation 19, Mittelsiederdestillation 20, Leichtsiederdestillation 21 und Partialkondensation 22. Alle diese Stufen werden in der Kolonne K4 durchgeführt. Im Auftriebsteil der Destillationskolonne K4 wird die Acrylsäure vom Lösungsmittel und den mittelsiedenden Nebenkomponenten, wie Maleinsäureanhydrid, frei destilliert (Stufe 21). Um den Leichtsiederanteil in der Acrylsäure zu reduzieren, wird vorteilhafterweise die Acrylsäure über Seitenabzug 42 aus der Kolonne K4 abgezogen. Diese Acrylsäure wird Roh-Acrylsäure genannt. Im Abtriebsteil der Kolonne K4 findet die Mittelsiederdestillation 20 und die Lösungsmittelgemischdestillation 19 statt. Das aus dem Sumpf der Kolonne K4 abgezogene Lösungsmittel wird über Leitung 43 wieder der Kolonne K2 zugeführt.

Am Kopf der Kolonne K4 wird dann nach einer Kondensation 22 ein an Leichtsiedern reicher Strom über Leitung 44 abgezogen. Da dieser Strom aber noch Acrylsäure enthält, wird er vorteilhafterweise nicht verworfen, sondern wieder in die Absorptionskolonne K2 rückgeführt und wieder der Aufarbeitung zugeführt, wie in Fig. 2 gezeigt.

Vorzugsweise wird das Sauerwasser aus Leitung 36, das noch Acrylsäure gelöst enthalten kann, bevor es gemäß dem erfindungsgemäßen Verfahren entsorgt wird, mit einem kleinen Teilstrom des nahezu Acrylsäure-freien Lösungsmittels aus dem Sumpf der Destillationskolonne K4 aus Leitung 40 extraktiv behandelt. Bei dieser Sauerwasser-Extraktion 18 wird ein Teil der Acrylsäure in das Lösungsmittel extrahiert und somit aus dem Sauerwasser zurückgewonnen. Das Sauerwasser extrahiert im Gegenzug die polaren mittelsiedenden Komponenten aus dem Lösungsmittelstrom und vermeidet damit eine Aufpegelung dieser Komponenten im Lösungsmittelkreislauf. Das nach der Extraktion zurückbleibende Sauerwasser wird als Nebenkomponentenstrom (2) über Leitung 45 entsorgt. Das bei der Sauerwasserextraktion entstehende, mit Acrylsäure beladene Lösungsmittel wird über Leitung 48 wieder zum Lösungsmittelstrom 43 zurückgeführt.

Besonders vorteilhaft ist es, den oben erwähnten Sauerwasserstrom aus Leitung 45 vor der Entsorgung so weit wie möglich vorzukonzentrieren. Dies kann z.B. dadurch geschehen, daß die Abwärme, die zum Abkühlen der Reaktionsgase in der Kolonne K2 abgeführt wird, dazu genutzt wird, das Sauerwasser einzudampfen und Wasserdampf abzutrennen. Dieser Wasserdampf kann z.B. der oben beschriebenen Verbrennungsanlage über den gasförmigen Nebenkomponentenstrom (1) hinzugegeben werden. Das verbleibende Sauerwasser mit besonders hohem Gehalt an brennbaren Stoffen liefert eine besonders große Menge an rückgewinnbarer Energie, die in Form von gespanntem Dampf gewonnen werden kann. Besonders vorteilhaft ist diese Energiegewinnung, wenn der erzeugte Dampf in der Anlage selbst verwendet werden kann, so z.B. zur Destillation der Acrylsäure in der Kolonne K4 oder in der Lösungsmitteldestillation und zum Antrieb der (Luft- und/oder Kreis-)Gasverdichter mittels Dampfturbine.

Die in Stufe (c) erhaltene Roh-Acrylsäure enthält, jeweils bezogen auf die Roh-Acrylsäure, vorzugsweise 98 bis 99,8 Gew.-%, insbesondere 98,5 bis 99,7 Gew.-%, Acrylsäure und 0,2 bis 2 Gew.-%, insbesondere 0,5 bis 1,5 Gew.-% Verunreinigungen, wie z.B. Essigsäure, Aldehyde und Maleinsäureanhydrid. Diese Acrylsäure kann, sofern die Anforderungen an ihre Reinheit nicht sehr hoch sind, gegebenenfalls bereits für eine Veresterung verwendet werden. Eine unter Umständen weitere notwendige Abtrennung der Nebenkomponenten von der so gewonnenen Rohsäure kann wahlweise durch Kristallisation, Destillation oder andere geeignete Trennoperationen erfolgen, je nach geforderter Qualität der Acrylsäure. Die so erhaltene Acrylsäure kann entweder direkt weiterverarbeitet werden oder weiteren chemischen Reaktionen wie z.B. einer Veresterung unterzogen werden.

Die Erfindung wird anhand des folgenden Beispiels, das ein bevorzugtes Ausführungsbeispiel darstellt, näher erläutert.

Die folgenden Angaben beziehen sich auf eine Acrylsäureproduktionsanlage, die nach dem in DE-A-2 136 396 beschriebenen Verfahren betrieben wird, wobei das acrylsäurehaltige Produkt nach der Synthese in einem hochsiedenden Lösungsmittel bestehend aus 80 Gew.-% Diphyl (Gemisch aus Diphenylether und Diphenyl) und 20 Gew.-% Dimethylphthalat (DMP) absorbiert wird. Das Reaktionsschema entspricht im wesentlichen dem in Fig. 2 beschriebenen Verfahren, wobei der Einfachkeit halber nur die Kolonnen/-Apparate K1 bis K4 erwähnt sind.

Aus einem Syntheseteil, der aus drei Reaktorstraßen mit je zwei Rohrbündelreaktoren als Oxidationsreaktoren besteht, erhält man als Reaktionsprodukt einen Gasstrom, dessen Mengenstrom von im Mittel folgender Zusammensetzung in Massen-% gemessen wurde:

| | |
|---|---|
| Acrylsäure | 9,3% |
| CO₂ | 3,2% |
| Stickstoff | 77,4% |
| Sauerstoff | 3,7% |
| Wasser | 3,8% |

Die im folgenden genannten Angaben beziehen sich jeweils auf diesen Reaktionsgasstrom, der im folgenden *Rohprodukt* genannt wird.

Dieses Rohprodukt wird in einem Vorquench (Direktkondensator) K1 mit 10 kg Diphyl/DMP pro Nm³ (Normkubikmeter) Rohprodukt als Lösungsmittel versetzt und daraufhin bei einer Gastemperatur von circa 170 °C in die Absorberkolonne K2 überführt. Die weitere Aufarbeitung geschieht wie weiter oben beschrieben.

Ein kleiner Seitenstrom des Lösungsmittels im Sumpf der Absorptionskolonne K2 von 0,5 bis 1 Gew.-% der über die Kolonne K1 umgepumpten Menge wird abgezogen und einer Entspannungsverdampfung (Flash-Verdampfung) in der Lösungsmittelaufarbeitung bei T = 150°C bis 200°C und p = 500 bis 200 mbar unterzogen. Hierbei fällt als Destillat ein reines Lösungsmittel an, während als Sumpf ein Nebenproduktstrom anfällt, der als Nebenkomponentenstrom (3) entsorgt wird. Die Elementarzusammensetzung dieses Nebenkomponentenstroms (3) beträgt im Mittel:

| | |
|---|---|
| C | 68,8% |
| H | 4,4% |
| O | 23,1% |
| N | 1,05% |
| S | 2,2% |

Hierbei stammen die Anteile an Stickstoff und Schwefel von dem eingesetzten Prozeßstabilisator Phenothiazin, der in der Destillationskolonne K4 zugegeben wird und durch die Kreislaufführung des Lösungsmittels ebenfalls in die Absorptionskolonne K2 gelangt. Der Nebenkomponentenstrom (3) ist ein schwärzlicher, fester bis zähfließender Stoff, der einen Stockpunkt von circa 100 °C besitzt.

Die in der Absorptionskolonne K2 nicht kondensierten Bestandteile (Inertgase, tiefstsiedende organische Inhaltsstoffe, Wasser CO, CO₂ etc.) des Rohprodukts werden in die Reaktoren als Kreisgas zurückgeführt, wobei ein Teilstrom abgezweigt wird und der Verbrennung als Nebenkomponentenstrom (1) zugeführt wird. Dieser abgezweigte Teilstrom besteht zu 90 Gew.-% aus Stickstoff, zu 4 Gew.-% aus Sauerstoff, zu 4 Gew.-% aus CO und CO₂, zu 1 Gew.-% aus Wasser sowie zu 1 Gew.-% aus organischen Bestandteilen. Ein weiterer Teilstrom des Kreisgases wird zum Ausstrippen von Leichtsiedern aus dem acrylsäurehaltigen Lösungsmittelstrom in der Kolonne K3 verwendet. Dieser dann mit leichtsiedenden Bestandteilen angereicherte Kreisgasstrom wird ebenfalls der Absorptionskolonne wieder zugeführt, um am Ende dieser Absorptionskolonne K2 wieder im Teilstrom des Kreisgases zu erscheinen, der rezirkuliert wird, oder in dem Teilstrom, der der Verbrennung zugeführt wird.

Der Stoffstrom, der die Absorptionskolonne K2 verläßt, wird im folgenden Schritt in einer Desorptionskolonne K3 mit dem oben beschriebenen Teilstrom des Kreisgases gestrippt. Nunmehr weitgehend von Leichtsiedern befreit, gelangt der Produkt- und Lösungsmittelstrom in die Destillationskolonne K4. In dieser wird das Produkt im Seitenabzug ausgeschleust. Das schwersiedende Lösungsmittel gelangt über den Sumpf wieder zurück auf die Absorptionskolonne K2. Die im Kopf der Kolonne K4 und im Kopfkondensator anfallenden Leichtsiederfraktionen werden ebenfalls in die Absorptionskolonne K2 zurückgeführt.

Das im oberen Kühlkreis der Absorptionskolonne K2 anfallende Wasser (Sauerwasser) wird nach einer Extraktion mit Kreislösungsmittel komplett der Verbrennung als Nebenkomponentenstrom (2) zugeführt. Dasselbe gilt für den ausgeschleusten Teilstrom des Kreisgases, der den Nebenkomponentenstrom (1) bildet.

Die Verbrennung der zu entsorgenden Nebenkomponentenströme erfolgt in einem Brenner des Typs Flammenverdampfungsbrenner, wie er in Fig. 1 gezeigt ist. Hierbei werden die einzelnen Stoffströme erst im Brennraum miteinander in Kontakt gebracht, wobei die Zuführung dieser Stoffströme mittels konzentrischer Auslaßringspalte erfolgt. Zusätzlich zu den oben genannten zu entsorgenden Nebenkomponentenströmen müssen hinzugeführt werden: Stützgas (vorzugsweise Erdgas), Verbrennungsluft und Zerstäuberluft.

Der Abhitzeteil der Brennkammer ist so aufgebaut, daß die heißen Abgase zunächst zur Erzeugung von hochgespanntem Wasserdampf eingesetzt werden und dann zur Überhitzung dieses 21 bar-Dampfes. Danach bedienen die heißen Abgase die zweite Stufe der zweistufigen Vorwärmung des zu entsorgenden Nebenkomponentenstroms (1) für die Verbrennung, daraufhin erwärmen sie die Verbrennungsluft, und als vorletztes beliefern sie die erste Stufe der zweistufigen Vorwärmung des Nebenkomponentenstrom (1) mit Energie. Die letztendlich verbleibende Energie wird je nach Einstellung der Anlage zur Erzeugung von niedergespanntem Dampf verwendet. Das bedeutet, daß die in der Anlage gewonnene Energie als Menge produzierten Dampfes gemessen werden kann.

In dieser Anlage wurden entsprechend dem erfindungsgemäßen Verfahren zwei Versuche durchgeführt, wobei bei dem ersten Versuch der Nebenkomponentenstrom (3) extern entsorgt wurde und beim zweiten Versuch dieser Nebenkomponentenstrom mitverbrannt wurde.

### 1. VERSUCH

Ein Reststoffanfall der Zusammensetzung, jeweils bezogen auf das Rohprodukt,

| | |
|---|---|
| gasförmiger Nebenkomponentenstrom (1) | 38,5% (V/V) |
| flüssig-wäßriger Nebenkomponentenstrom (2) | 3,7% (m/m) |
| flüssig-pastöser, organischer Nebenkomponentenstrom (3) | 0,3% (m/m) |

wurde entsorgt, indem die gasförmigen und flüssig-wäßrigen Ströme (1) und (2) rückstandsfrei verbrannt wurden und der Komponentenstrom (3) extern entsorgt wurde.

Bei der Verbrennung betrug die Temperatur am Ende der Brennkammer etwa 904°C. Es wurde eine Erhöhung der Produktion hochgespannten Dampfes um 22,4% gegenüber einem Nullversuch, der nur mit Stützgas (anstelle der zu entsorgenden Nebenkomponentenströme) durchgeführt wurde, gefunden.

### 2. VERSUCH

Ein Reststoffanfall der Zusammensetzung, jeweils bezogen auf das Rohprodukt,

| | |
|---|---|
| gasförmiger Nebenkomponentenstrom (1) | 38,500% (V/V) |
| flüssig-wäßriger Nebenkomponentenstrom (2) | 3,7% (m/m) |
| flüssig-pastöser, organischer Nebenkomponentenstrom (3) | 0,4% (m/m) |

wurde rückstandsfrei entsorgt, indem alle drei Stoffströme der oben beschriebenen Verbrennung zugeführt wurden. Da hierbei der Nebenkomponentenstrom (3) ebenfalls mitverbrannt wurde, wurde eine Düse mit einem weiteren Zuführungsringspalt im Brennersystem verwendet.

Es wurde eine Erhöhung der Dampfproduktion von 24,6% gegenüber dem Nullversuch (Verbrennung nur mit Stützgas) gemessen.

Die vorliegenden Versuche zeigen somit, daß das erfindungsgemäße Verfahren eine rückstandsfreie Entsorgung der bei der Acrylsäure-Herstellung anfallenden Nebenkomponentenströme gewährleistet.

## Patentansprüche

1. Verfahren zur Entsorgung von bei der Acrylsäure- oder Methacrylsäure-Herstellung anfallenden Nebenkomponenten, **dadurch gekennzeichnet, daß** gasförmige leichtsiedende Nebenkomponenten (1) verbrannt werden, wobei in Wasser gelöste leicht- oder mittelsiedende Nebenkomponenten (2) in die Verbrennungsstufe der Nebenkomponenten (1) zugeführt werden, wobei die leichtsiedenden Nebenkomponenten einen niedrigeren Siedepunkt als die Acrylsäure oder Methacrylsäure besitzen und die mittelsiedenden Nebenkomponenten in etwa den gleichen Siedepunkt wie die Acrylsäure oder Methacrylsäure besitzen, wobei die Acrylsäure oder Methacrylsäure hergestellt wird durch
(a) katalytische Gasphasenoxidation (12) von Propen oder Isobuten und/oder Acrolein oder Methacrolein,
(b) Absorption (14) des in Stufe (a) entstehenden Reaktionsprodukts in einem Lösungsmittel und
(c) Abtrennung der Acrylsäure oder Methacrylsäure aus dem beladenen Lösungsmittel der Stufe (b) durch Extraktion und/oder Destillation (19, 20, 21, 22),
wobei vor der Absorption (14) in Stufe (b) ein Teil des Lösungsmittels verdampft wird (13) und von dem verbleibenden flüssigen Lösungsmittel ein Teil, gegebenenfalls nach Lösungsmittelverdampfung, als schwersiedende Nebenkomponente (3) entsorgt wird, und
nach der Absorption (14) des Reaktionsprodukts in Stufe (b) verbleibendes, nicht absorbiertes Reaktionsprodukt abgekühlt wird (15), wobei das erhaltene wäßrige Kondensat, gegebenenfalls nach anschließender Extraktion, als Nebenkomponente (2) und wenigstens ein Teil des erhaltenen Gasstroms als Nebenkomponente (1) entsorgt werden.

2. Verfahren nach Anspruch 1, **dadurch gekennzeichnet, daß** die in Wasser gelösten leicht- oder mittelsiedenden Nebenkomponenten (2) zusammen mit mit mit niedrigviskosem Lösungsmittel behandelten schwersiedenden Nebenkomponenten (3) in die Verbrennungsstufe der Nebenkomponenten (1) zugeführt werden, wobei die schwersiedenden Nebenkomponenten einen höheren Siedepunkt also die Acrylsäure oder Methacrylsäure besitzen.

3. Verfahren nach Anspruch 1 oder 2, **dadurch gekennzeichnet, daß** die leichtsiedenden Nebenkomponenten (1) Kohlenmonoxid, Kohlendioxid, Stickstoff und/oder bei der Acrylsäure- oder Methacrylsäure-Herstellung nicht umgesetzte Edukte enthalten.

4. Verfahren nach einem der Ansprüche 1 bis 3, **dadurch gekennzeichnet, daß** die Nebenkomponenten (2) Essigsäure, Maleinsäure, Fumarsäure und/oder Formaldehyd enthalten.

5. Verfahren nach einem der vorhergehenden Ansprüche, **dadurch gekennzeichnet, daß** die schwersiedenden Nebenkomponenten (3) Acrylsäurepolymerisate oder Methacrylsäurepolymerisate enthalten.

6. Verfahren nach einem der vorhergehenden Ansprüche, **dadurch gekennzeichnet, daß** die Verbrennung in einer Brennkammer mit einem Stützgas- oder Mehrstoffbrenner durchgeführt wird.

7. Verfahren nach einem der vorhergehenden Ansprüche, **dadurch gekennzeichnet, daß** die Verbrennung bei Temperaturen von 800 bis 1000°C durchgeführt wird.

8. Verfahren nach einem der vorhergehenden Ansprüche, **dadurch gekennzeichnet, daß** bei der Verbrennung zusätzlich Erdgas und/oder Luft zugeführt werden.

9. Verwendung einer Brennkammer mit einem oder mehreren Stützgas- oder Mehrstoffbrennern zur Durchführung des Verfahrens gemäß einem der Ansprüche 1 bis 8.

## Claims

1. A process for disposing of secondary components obtained in the preparation of acrylic acid or methacrylic acid, wherein gaseous low-boiling secondary components (1) are incinerated, low-boiling or medium-boiling secondary components (2) dissolved in water being fed to the incineration stage of the secondary components (1), the low-boiling secondary components having a lower boiling point than acrylic acid or methacrylic acid and the medium-boiling secondary components having about the same boiling point as acrylic acid or methacrylic acid, wherein the acrylic acid or methacrylic acid is prepared by
(a) catalytic gas-phase oxidation (12) of propene or isobutene and/or acrolein or methacrolein,
(b) absorption (14) of the reaction product formed in stage (a) in a solvent and
(c) isolation of the acrylic acid or methacrylic acid from the laden solvent of stage (b) by extraction and/or distillation (19, 20, 21, 22),
a part of the solvent being vaporized (13) before the absorption (14) in stage (b) and a part of the remaining liquid solvent being disposed of as high-boiling secondary component (3), if required after solvent vaporization, and unabsorbed reaction product remaining after the absorption (14) of the reaction product in stage (b) being cooled (15), the aqueous condensate obtained being disposed of, if required after subsequent extraction, as secondary component (2) and at least a part of the gas stream obtained being disposed of as secondary component (1).

2. A process a claimed in claim 1, wherein the low-boiling or medium-boiling secondary components (2) dissolved in water are fed to the incineration stage of the secondary components (1) together with high-boiling secondary components (3) treated with low-viscosity solvent, the high-boiling secondary components having a higher boiling point than acrylic acid or methacrylic acid.

3. A process as claimed in claim 1 or 2, wherein the low-boiling secondary components (1) contain carbon monoxide, carbon dioxide, nitrogen and/or starting materials not converted in the preparation of acrylic acid or methacrylic acid.

4. A process as claimed in any of claims 1 to 3, wherein the secondary components (2) contain acetic acid, maleic acid, fumaric acid and/or formaldehyde.

5. A process as claimed in any of the preceding claims, wherein the high-boiling secondary components (3) contain acrylic acid polymers or methacrylic acid polymers.

6. A process as claimed in any of the preceding claims, wherein the incineration is carried out in a combustion chamber having a gas-assisted burner or multifuel burner.

7. A process as claimed in any of the preceding claims, wherein the incineration is carried out at from 800 to 1000°C.

8. A process as claimed in any of the preceding claims, wherein the natural gas and/or air are additionally fed in during the incineration.

9. The use of a combustion chamber having one or more gas-assisted burners or multifuel burners for carrying out the process as claimed in any of claims 1 to 8.

## Revendications

1. Procédé pour l'élimination des composants secondaires produits au cours de la production de l'acide acrylique ou de l'acide méthacrylique, **caractérisé en ce que** les composants secondaires gazeux à bas point d'ébullition (1) sont brûlés et que les composants secondaires à point d'ébullition bas ou moyen (2) dissous dans l'eau sont amenés dans l'étape de combustion des composants secondaires (1), les composants secondaires à bas point d'ébullition ayant un point d'ébullition inférieur à celui de l'acide acrylique ou de l'acide méthacrylique et les composants secondaires à point d'ébullition moyen ayant un point d'ébullition presque identique à celui de l'acide acrylique ou de l'acide méthacrylique, et l'on produit l'acide acrylique ou méthacrylique par
(a) oxydation en phase vapeur catalytique (12) du propène ou du isobutène et/ou de l'acroléine ou méthacroléine,
(b) absorption (14) du produit réactionnel provenant de l'étape (a) dans un solvant, et
(c) séparation de l'acide acrylique ou méthacrylique du solvant chargé provenant de l'étape (b) par extraction et/ou distillation (19, 20, 21, 22),
dans lequel, on évapore une partie du solvant (13) avant l'absorption (14) dans l'étape (b) et on élimine une partie du solvant liquide restant, éventuellement après une évaporation du solvant, en tant que composant secondaire d'ébullition difficile (3), et
on refroidit (15) le produit réactionnel qui n'a pas été absorbé et qui est resté dans l'étape (b) après l'absorption (14), et on élimine le produit de condensation aqueux ainsi obtenu, éventuellement après une extraction ultérieure, en tant que composant secondaire (2), et le courant de gaz obtenu au moins partiellement en tant que composant secondaire (1).

2. Procédé selon la revendication 1, **caractérisé en ce que** l'on amène les composants secondaires à point d'ébullition bas ou moyen (2) dissous dans l'eau, conjointement avec les composants secondaires d'ébullition difficile, qui sont traités avec des solvants de faible viscosité, dans l'étape de combustion des composants secondaires (1); les composants secondaires d'ébullition difficile ayant un point d'ébullition supérieur à celui de l'acide acrylique ou de l'acide méthacrylique.

3. Procédé selon la revendication 1 ou 2, **caractérisé en ce que** les composants secondaires à bas point d'ébullition (1) comprennent du monoxyde de carbone, du dioxyde de carbone, de l'azote et/ou des éduits non transformés lors de la production de l'acide acrylique ou de l'acide méthacrylique.

4. Procédé selon l'une quelconque des revendications 1 à 3, **caractérisé en ce que** les composants secondaires (2) comprennent de l'acide acétique, de l'acide maléique, de l'acide fumarique et/ou de l'aldéhyde formique.

5. Procédé selon l'une quelconque des revendications précédentes, **caractérisé en ce que** les composants secondaires d'ébullition difficile (3) comprennent des polymères d'acide acrylique ou méthacrylique.

6. Procédé selon l'une quelconque des revendications précédentes, **caractérisé en ce que** l'on réalise la combustion dans une chambre de combustion muni d'un brûleur à gaz ou d'un brûleur multicarburant.

7. Procédé selon l'une quelconque des revendications précédentes, **caractérisé en ce que** l'on réalise la combustion à des températures de 800°C à 1000°C.

8. Procédé selon l'une quelconque des revendications précédentes, **caractérisé en ce que** l'on ajoute du gaz naturel et/ou de l'air pendant la combustion.

9. Utilisation d'une chambre de combustion muni d'un ou de plusieurs brûleurs à gaz ou multicarburants pour la réalisation du procédé selon l'une quelconque des revendications 1 à 8.
